# EUROPÄISCHE PATENTANMELDUNG

(11) **EP 3 636 200 A1**
(43) Veröffentlichungstag der Anmeldung: **15.04.2020**
(21) Anmeldenummer: 19202864.5
(22) Anmeldetag: 11.10.2019
(51) Int. Cl.: A61B 90/00, A61B 17/62

(54) **RÖNTGENMARKER FÜR EIN MEDIZINISCHES INSTRUMENT UND KIT**

(30) Priorität: 12.10.2018 DE 102018125357
(71) Anmelder: OT Medizintechnik GmbH, 80639 München (DE)
(72) Erfinder: Schreiber, Ulrich, 80639 München (DE)
(74) Vertreter: Bobbert & Partner Patentanwälte PartmbB

(57) **Zusammenfassung**

Die Erfindung betrifft einen Röntgenmarker (100) für ein medizinisches Instrument, insbesondere zum Einstecken in Öffnungen (301) eines Ringfixateurs (300), aufweisend einen röntgendichten Abschnitt (120). Weiterhin betrifft die Erfindung ein Kit mit wenigstens einem oder mehreren erfindungsgemäßen Röntgenmarkern (100) sowie wenigstens einer Löcherleiste (200) aus einem nicht röntgendichten Material oder ein solches aufweisend, wobei die Löcherleiste (200) eine Vielzahl von Sacklöchern oder Durchgangslöchern (201) aufweist, deren Durchmesser es erlaubt, einen der Röntgenmarker (100) zumindest abschnittsweise aufzunehmen.

## Beschreibung

Die Erfindung bezieht sich auf einen Röntgenmarker gemäß Anspruch 1 zur Verwendung mit einem medizinischen Instrument sowie auf ein Kit gemäß Anspruch 11.

Nach einer, insbesondere traumatischen, Fraktur eines Knochens ist es häufig erforderlich, die Frakturenden erneut aufeinander auszurichten oder in Stellung zueinander zu bringen. Hierfür können Haltesysteme am Knochen, sogenannte Fixateurs Externes, verwendet werden. Zur Ausrichtung des Haltesystems und zur radiologischen Überwachung des Heilungsprozesses können Marker mit dem Haltesystem verbunden werden, um z. B. Strukturen von Interesse während einer Röntgenkontrolle besser darstellen oder erkennen zu können. Diese Marker werden in der Praxis als Röntgenmarker bezeichnet.

Es ist die Aufgabe der vorliegenden Erfindung, einen weiteren Röntgenmarker und ein Kit anzugeben.

Die erfindungsgemäße Aufgabe kann gelöst werden durch den Röntgenmarker mit den Merkmalen des Anspruchs 1 und durch das Kit mit den Merkmalen des Anspruchs 11.

Somit weist der erfindungsgemäße Röntgenmarker wenigstens einen, optional röntgendichten, Abschnitt auf. Der röntgendichte Abschnitt dient dazu, für Röntgenstrahlen weitgehend undurchlässig zu sein. Somit kann die Position dieses röntgendichten Abschnitts auf z. B. einem Röntgenbild eindeutig lokalisiert werden. Damit kann die Position des Röntgenmarkers und damit die Position eines mit dem Röntgenmarker verbundenen Instruments, oder eines Abschnitts hiervon, schnell und einfach mittels, oder auf, einer Röntgenaufnahme erfasst werden.

Der Röntgenmarker ist insbesondere zum Einstecken in Öffnungen eines Ringfixateurs vorgesehen und kann entsprechend konfiguriert, z. B. dimensioniert, sein.

Das erfindungsgemäße Kit weist wenigstens einen oder mehrere erfindungsgemäße Röntgenmarker auf. Weiterhin weist das Kit wenigstens eine Löcherleiste auf (diese kann ein Löcherabschnitt sein und/oder als Lochleiste bezeichnet werden, weshalb alles, was hierin zur Löcherleiste ausgeführt ist, auch auf den Löcherabschnitt zutrifft und umgekehrt; diese Begriffe sind hierin austauschbar), der optional aus einem nicht-röntgendichten Material besteht oder ein solches Material aufweist. Die Löcherleiste weist eine Vielzahl von Sacklöchern oder Durchgangslöchern auf, deren Durchmesser es erlauben, jeweils einen der erfindungsgemäßen Röntgenmarker zumindest abschnittsweise aufzunehmen.

Bei allen vorangegangenen und bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Vorteilhafte Weiterentwicklungen der vorliegenden Erfindung sind jeweils Gegenstand von Unteransprüchen und Ausführungsformen.

Wenn hierin von einer Ausführungsform die Rede ist, so ist damit eine exemplarische, erfindungsgemäße Ausführungsform zu verstehen, die nicht als beschränkend zu verstehen ist.

Erfindungsgemäße Ausführungsformen können eines oder mehrere der vorstehend und/oder im Folgenden genannten Merkmale in jeder beliebigen Kombination aufweisen, sofern eine solche Kombination für den Fachmann nicht als technisch unmöglich zu erkennen ist.

In einigen Ausführungsformen bestehen ein, mehrere oder alle Teile oder Abschnitte des Röntgenmarkers aus einer Kobalt-Chrom-Legierung (CoCr) oder weisen eine solche Legierung auf.

In manchen Ausführungsformen bestehen ein, mehrere oder alle Teile oder Abschnitte des Röntgenmarkers aus Titan, insbesondere Titan Grad 5 oder weisen Titan auf.

In einigen Ausführungsformen bestehen ein, mehrere oder alle Teile oder Abschnitte des Röntgenmarkers ganz oder teilweise aus Pyrocarbon und/oder Keramik.

In manchen Ausführungsformen bestehen ein, mehrere oder alle Teile oder Abschnitte des Röntgenmarkers teilweise oder ganz aus einem Kunststoff (insbesondere Polyethylen, insbesondere hochvernetztes Polyethylen, insbesondere mit Vitamin E) oder umfasst einen solchen.

In manchen Ausführungsformen weist der röntgendichte Abschnitt zumindest einen zylindrischen Abschnitt auf. Der zylindrische Abschnitt kann ein Hohlzylinder sein oder aus einem Vollmaterial bestehen.

In manchen Ausführungsformen weist der röntgendichte Abschnitt wenigstens einen Abschnitt in Form einer Kugel oder eines Kugelteilabschnitts, optional auch einer anderen Form, auf, was die Lage des röntgendichten Abschnitts im Raum z. B. aus einem Röntgenbild heraus besser erkennen lässt. Dieser Abschnitt steht in einigen Ausführungsformen über den zylinderförmigen Abschnitt des röntgendichten Abschnitts radial und/oder in Längsrichtung (axial) über.

In manchen Ausführungsformen weist der röntgendichte Abschnitt wenigstens einen Abschnitt in Form von wenigstens zwei Kugeln oder wenigstens zwei Kugelteilabschnitten, optional auch einer anderen Form, auf. Dieser Abschnitt steht in einigen Ausführungsformen über den zylinderförmigen Abschnitt des röntgendichten Abschnitts radial und/oder in Längsrichtung über.

In manchen Ausführungsformen weist der Röntgenmarker einen nicht-röntgendichten Abschnitt auf. Der nicht-röntgendichte Abschnitt kann den röntgendichten Abschnitt teilweise oder vollständig umgeben. Beispielsweise kann der röntgendichte Abschnitt als zylindrischer Stab in eine Bohrung oder Öffnung des nicht-röntgendichten Abschnitts eingeschoben werden. Die Bohrung oder Öffnung kann eine Sacklochbohrung oder eine Durchgangsbohrung sein. Der röntgendichte Abschnitt und der nicht-röntgendichte Abschnitt können im Gebrauch des Röntgenmarkers konzentrisch zueinander angeordnet sein. Der röntgendichte Abschnitt kann in einem Abschnitt eine zylindrische Form aufweisen und an einem längsseitigen Ende optional einen runden, kugelförmigen, ovalen, eckigen oder anders geformten Aufsatz bzw. Ansatz aufweisen. Damit kann die Lage des Röntgenmarkers oder eines den Röntgenmarker aufweisenden, medizinischen Instruments bei einer Röntgendurchleuchtung vorteilhaft einfach bestimmt werden.

In manchen Ausführungsformen läuft der nicht-röntgendichte Abschnitt und/oder der röntgendichte Abschnitt zumindest abschnittsweise konisch zu. Dadurch kann ein erneut lösbares Verbinden des Röntgenmarkers, beispielsweise mittels Einschiebens in einen aufnehmenden oder umgebenden Abschnitt, vorteilhaft einfach erfolgen.

In manchen Ausführungsformen weist der erfindungsgemäße Röntgenmarker einen Kragen auf, der, falls vorgesehen, über den konisch zulaufenden Abschnitt oder andere Abschnitte eines Grundkörpers vorzugsweise radial, also senkrecht zu einer Längsachse des Röntgenmarkers, überstehen kann.

In manchen Ausführungsformen weist der erfindungsgemäße Röntgenmarker wenigstens eine Lippe auf, die einen Abschnitt des Röntgenmarkers, z. B. dessen Grundkörper, nach radial abschließt. Die Lippe kann vorteilhaft als Kragen oder Absatz bezeichnet werden. Die Lippe kann als Positionierhilfe und/oder als axiale Rast- und Fixierhilfe für eine Löcherleiste oder auf oder innerhalb dieser verwendet werden. Die Lippe kann in Umfangsrichtung des Röntgenmarkers geschlossen oder umlaufend sein. Die Lippe kann einen geringeren Außendurchmesser haben als der weiter oben genannte Kragen, der den Röntgenmarker optional zu einem Ende hiervon axial abschließt.

Der Abstand zwischen Lippe und Kragen beträgt in einigen Ausführungsformen exakt oder im Wesentlichen (z. B. +/- 5 % bis 20%) der Dicke einer Löcherleiste.

Der Abstand zwischen einer Lippe und einer benachbarten Lippe beträgt in einigen Ausführungsformen exakt oder im Wesentlichen (z. B. +/- 5 % bis 20%) der Dicke einer Löcherleiste.

In manchen Ausführungsformen weist der Röntgenmarker einen radialen Außendurchmesser oder Maximaldurchmesser in einem Bereich zwischen 5 mm und 20 mm, vorzugsweise zwischen 5 mm und 15 mm, besonders bevorzugt zwischen 7 mm und 12 mm auf.

Das erfindungsgemäße Kit kann optional wenigstens einen Fixateur oder Ringfixateur mit der Form eines Teils eines Kreises oder eines Teils einer Ellipse aufweisen. Der Fixateur oder Ringfixateur kann eine Vielzahl von Sacköffnungen oder Durchgangsöffnungen aufweisen. Die Sacklöcher oder Durchgangslöcher, oder manche hiervon, weisen optional einen Durchmesser auf, der es erlaubt, einen der erfindungsgemäßen Röntgenmarker zumindest abschnittsweise darin aufzunehmen.

Das erfindungsgemäße Kit weist in einigen Ausführungsformen einen Fixateur oder Ringfixateur (kurz: Fixateur) auf, der keine entlang seines Umfangs geschlossene Kreisform oder keine entlang seines Umfangs geschlossene Ellipse oder andere geometrische Form (Rechteck, Quadrat, usw.) darstellt. Zudem weist das Kit in diesen Ausführungsformen eine oder mehrere Löcherleisten auf, die mittels Röntgenmarker (und vorzugsweise bereits unter Einsatz nur der Röntgenmarker, also ohne weitere Verbinder einsetzen zu müssen) derart miteinander und/oder mit dem Fixateur lösbar verbunden oder verbindbar sind, dass ein geschlossener Umfang Kombination aus Fixateur oder Ringfixateur und Löcherleisten erzielt ist.

Wenn hierin davon die Rede ist, dass ein Abschnitt, Element oder Bauteil röntgendicht ist, so ist von der vorliegenden Erfindung auch umfasst, dass diese Struktur optional nichtröntgendicht ist. Ebenso gilt, dass, wenn hierin davon die Rede ist, dass ein Abschnitt, Element oder Bauteil nichtröntgendicht ist, von der vorliegenden Erfindung auch umfasst ist, dass diese Komponente oder Struktur optional röntgendicht ist. Dies betrifft insbesondere jede der in den Ansprüchen genannten und jede der in den Figuren gezeigten Strukturen.

In einigen Ausführungsformen weist die Löcherleiste eine Vielzahl von Sacklöchern oder Durchgangslöchern auf, die als Langlöcher ausgestaltet sind. Langlöcher sind vorzugweise längliche Bohrung, Nuten oder Öffnungen und haben schmale Seiten und Längsseiten. Die schmalen Seiten sind zumeist durch Halbkreise abgeschlossen, deren Durchmesser der Breite des Langlochs entsprechen. Die Längsseiten des Langloches verlaufen parallel zueinander. Langlöcher erlauben vorzugsweise eine freiere Orientierung der Löcherleisten zueinander und in Relation zum Ringfixateur oder Fixateur.

In einigen Ausführungsformen sind einige oder alle der folgenden Vorteile erzielbar.

Die erfindungsgemäßen Röntgenmarker können vorteilhaft zur intraoperativen Navigation, Ausrichtung und Orientierung von sogenannten Fixateurs Externes, insbesondere von Ringfixateuren, verwendet werden. Aufgrund des röntgendichten Abschnitts des Röntgenmarkers kann die Sichtbarkeit z. B. des Fixateur Externe oder von Abschnitten hiervon während einer Röntgenkontrolle verbessert werden.

Der Fixateur oder Ringfixateur kann mittels der Löcherleisten auf einfache Weise und optional ohne Werkzeug mit hoher Genauigkeit zu einem geschlossenen Umfang vervollständigt und dieser auch wieder geöffnet werden. Hierbei kann erfindungsgemäß Zeit eingespart werden.

Die erfindungsgemäße Lösung erlaubt eine Ausrichtung von Knochenfragmenten zueinander unter Verwendung von nur wenigen Bauteilen, die kostengünstig und unter einfachstem Design zu fertigen sind.

Der optional sich konisch nach unten (also in Einführrichtung des Röntgenmarkers oder in Richtung auf das einem optionalen Kragen gegenüberliegende Ende) verjüngende Abschnitt erlaubt es vorteilhaft, den Röntgenmarker in Öffnungen einzustecken, deren Durchmesser in gewissem Maße variieren können. Auf diese Weise kann der Röntgenmarker gemeinsam mit Ringfixateuren verschiedener Hersteller oder Ausführungen verwendet werden.

Das optionale Vorsehen von Wulsten, Rippen, Lippen oder Ringen erlaubt das Erzielen eines Passsitzes oder eines Presssitzes auch unter Aufbringung von nur geringer Kraft zum Einführen oder Einstecken des Röntgenmarkers in die ausgewählte Öffnung. Weiterhin kann der Röntgenmarker vorteilhaft einfach, insbesondere mit geringem Kraftaufwand, wieder aus der Öffnung herausgezogen und entfernt werden.

Das Vorsehen von wenigstens zwei, insbesondere in Längsrichtung des Röntgenmarkers, voneinander beabstandeten Wulsten oder Ringen erlaubt das Zentrieren des Röntgenmarkers in der Öffnung und/oder in zwei Öffnungen zur selben Zeit.

Im Folgenden wird der erfindungsgemäße Röntgenmarker anhand bevorzugter Ausführungsformen beschrieben. Die Erfindung ist jedoch nicht auf diese Ausführungsformen beschränkt. In den Figuren gilt:
- **Fig. 1**: zeigt den erfindungsgemäßen Röntgenmarker einer Ausführungsform von vorne sowie im Längsschnitt;
- **Fig. 2**: zeigt den erfindungsgemäßen Röntgenmarker der Fig. 1 im Längsschnitt in Vergrößerung;
- **Fig. 3**: zeigt den in Fig. 2 mit B bezeichneten Abschnitt vergrößert;
- **Fig. 4**: zeigt den Röntgenmarker der vorangegangenen Figuren in einer Perspektive von oben;
- **Fig. 5**: zeigt eine mögliche Ausgestaltung des röntgendichten Abschnitts eines erfindungsgemäßen Röntgenmarkers in perspektivischer Ansicht;
- **Fig. 6**: zeigt den röntgendichten Abschnitt der Fig. 5 von der Seite;
- **Fig. 7**: zeigt eine mögliche Ausgestaltung des nichtröntgendichten Löcherabschnitts mit Öffnungen von der Seite;
- **Fig. 8**: zeigt eine mögliche Ausgestaltung des nichtröntgendichten Löcherabschnitts mit Öffnungen von vorne;
- **Fig. 9**: zeigt eine mögliche Ausgestaltung des nichtröntgendichten Löcherabschnitts mit Öffnungen in perspektivischer Ansicht;
- **Fig. 10**: zeigt zwei mit einem erfindungsgemäßen Röntgenmarker verbundene Löcherabschnitte in einer Aufsicht;
- **Fig. 11**: zeigt die verbundenen Löcherabschnitte der Fig. 10 in einer Schnittebene A-A in einer Seitenansicht;
- **Fig. 12**: zeigt einen Ausschnitt der Fig. 11 mit dem erfindungsgemäßen Röntgenmarker;
- **Fig. 13**: zeigt die zwei mit dem erfindungsgemäßen Röntgenmarker verbundenen Löcherabschnitte der Fig. 10 in einer perspektivischen Ansicht;
- **Fig. 14**: zeigt den erfindungsgemäßen Röntgenmarker in verschiedenen Ansichten mit rein exemplarischen Maßangaben;
- **Fig. 15**: zeigt eine Ausführungsform des erfindungsgemäßen Röntgenmarkers mit zwei stirnseitig gegenüberliegenden, konischen Abschnitten in einer perspektivischen Ansicht;
- **Fig. 16**: zeigt die Ausführungsform des erfindungsgemäßen Röntgenmarkers der Fig. 15 mit rein exemplarischen Maßangaben in verschiedenen Ansichten;
- **Fig. 17**: zeigt den nicht-röntgendichten Löcherabschnitt mit rein exemplarischen Maßangaben in verschiedenen Ansichten;
- **Fig. 18**: zeigt zwei Löcherabschnitte und fünf erfindungsgemäße Röntgenmarker als Einzelteile;
- **Fig. 19**: zeigt einen Ringfixateur, der mit den Einzelteilen der Fig. 18 verbunden ist, in einer Seitenansicht;
- **Fig. 20**: zeigt eine Aufsicht von zwei unterschiedlich großen, nebeneinander angeordneten Ringfixateuren, die jeweils mit Löcherabschnitten und Röntgenmarkern verbunden sind;
- **Fig. 21**: zeigt eine exemplarische, chirurgische Anwendung an einem Patienten einer Anordnung mit Ringfixateuren, Löcherabschnitten und erfindungsgemäßen Röntgenmarkern;
- **Fig. 22**: zeigt die Anordnung der Fig. 21 ohne Patienten;
- **Fig. 23**: zeigt eine weitere Anordnung von Ringfixateuren während einer Beinoperation;
- **Fig. 24**: zeigt das Anpassen eines Ringfixateurs an ein Bein eines Patienten, wobei die Löcherabschnitte noch nicht mittels erfindungsgemäßer Röntgenmarker miteinander verbunden sind;
- **Fig. 25**: zeigt das Anpassen des Ringfixateurs der Fig. 24 in einem zweiten Schritt; und
- **Fig. 26**: zeigt das Anpassen des Ringfixateurs der Fig. 24 und 25 in einem dritten Schritt, wobei die Löcherabschnitte mittels erfindungsgemäßer Röntgenmarker miteinander verbunden sind.

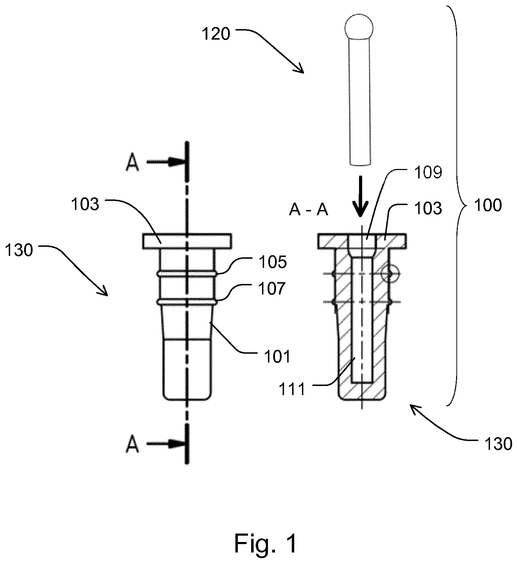

**Fig. 1** zeigt einen exemplarisch ausgestalteten erfindungsgemäßen Röntgenmarker 100 im nicht-montierten Zustand mit einem röntgendichten Abschnitt 120 und einem nicht-röntgendichten Abschnitt 130. Der nicht-röntgendichten Abschnitt 130 ist von vorne (links) sowie im Längsschnitt (rechts) dargestellt.

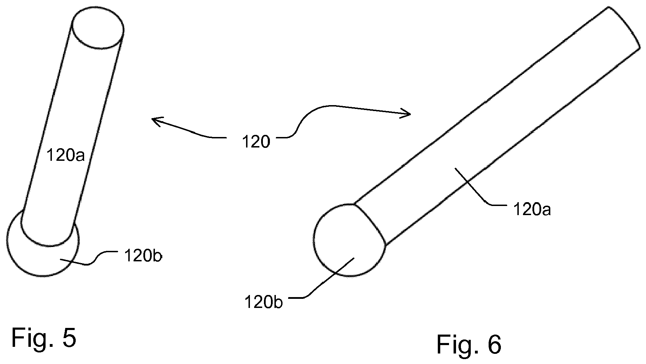

Der Grundkörper des Röntgenmarkers 100 hat, wie in Fig. 1 zu erkennen ist, eine optionale Öffnung 109 zu einem Innenraum 111, in den z. B. der in Fig. 5 und 6 im Detail gezeigte röntgendichte Abschnitt 120 vorzugsweise lösbar eingeschoben werden kann. Zur Montage des Röntgenmarkers 100 kann der röntgendichten Abschnitt 120 ganz oder teilweise durch eine Öffnung 109 in einen Innenraum 111 des nicht-röntgendichten Abschnitt 130 eingeführt werden. Dieses Einführen wird durch den Pfeil unterhalb des röntgendichten Abschnitt 120 angedeutet.

Der Röntgenmarker 100 weist einen Grundkörper mit einem optionalen konischen Abschnitt 101 und einen optionalen, an den Grundkörper angesetzten, Kragen 103 auf. Der konische Abschnitt 101 verjüngt sich hier in Richtung auf das der o. g. Öffnung 109 gegenüberliegende Ende des nicht-röntgendichten Abschnitts 130. Der optionale Kragen 103 oder radiale, umlaufende Überstand kann den Abschnitt 130 in Längsrichtung abschließen (also dessen Ende in einer Längsrichtung ausmachen) oder ein Aufsatz auf den Grundkörper sein, wie in Fig. 1 gezeigt.

Zwischen konischem Abschnitt 101 und jedem der beiden Enden des nicht-röntgendichten Abschnitts 130 kann sich optional jeweils ein nicht-konischer Abschnitt anschließen.

Zwei oder mehr Lippen oder Ringe 105 und 107, die den Grundkörper des röntgendichten Abschnitts 130 des Röntgenmarkers 100 entlang dessen Umfangs oder Teilen hiervon umgeben und dessen - auf den die Lippen oder Ringe 105 und 107 tragenden Bereich bezogen - maximale radiale Ausdehnung (mit Ausnahme des optionalen Kragens 103) ausmachen, können optional vorgesehen sein. Die Lippen oder Ringe 105 und 107 können in einem nicht-konischen Abschnitt vorgesehen sein. Sie können zwischen dem optionalen Kragen 103 und dem optionalen konischen Abschnitt 101 vorgesehen sein. Sie können gleiche Umfänge aufweisen.

Die Öffnung 109 oder der Innenraum 111 kann eine Sacköffnung oder eine Durchgangsöffnung sein.

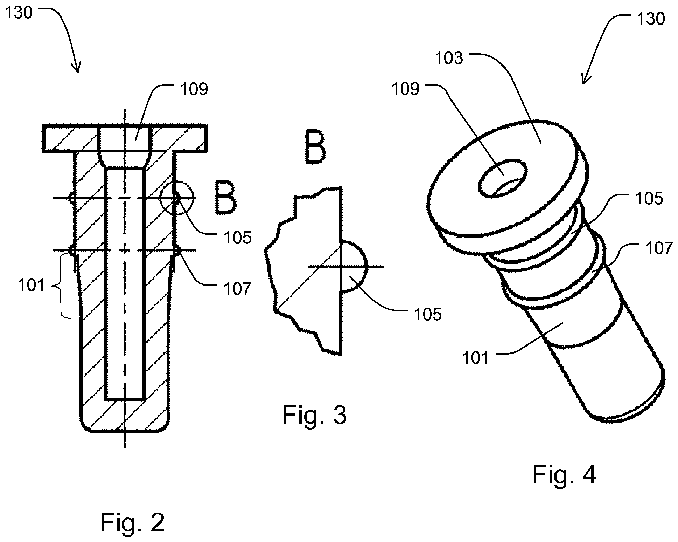

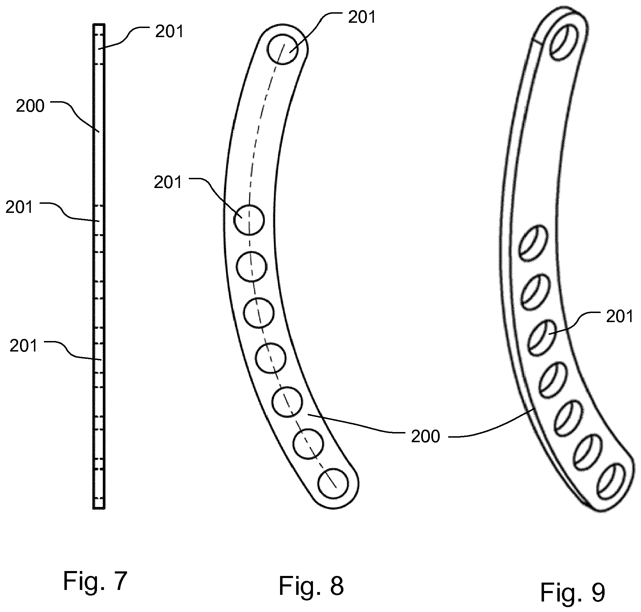

**Fig. 2** zeigt den nicht-röntgendichten Abschnitt 130 des erfindungsgemäßen Röntgenmarkers 100 der Fig. 1 im Längsschnitt in Vergrößerung. **Fig. 3** zeigt den in Fig. 2 mit B bezeichneten Abschnitt vergrößert. Mittels der optionalen Lippen 105 und 107 können beispielsweise Löcherleisten 200, wie sie in den Fig. 7 bis 13 gezeigt sind, miteinander verbunden, fixiert oder verrastet werden. Die Lippen 105 und 107 sind vorzugsweise einstückig mit dem nicht-röntgendichten Abschnitt 130 hergestellt, beispielsweise in einem Spritzgussverfahren.

**Fig. 4** zeigt den nicht-röntgendichten Abschnitt 130 des Röntgenmarkers 100 der vorangegangenen Figuren in Perspektive von oben. Der unterhalb der beiden optionalen Lippen 105 und 107 angeordnete, optionale, konische Bereich 101 ist in dieser Darstellung bezeichnet. Unterhalb des konischen Bereichs 101 schließt sich optional ein zylindrischer Bereich bis zum stirnseitigen Ende an. Am oberen Ende des nicht-röntgendichten Abschnitts 130 sind der Kragen 103 und die Öffnung 109 zum Einführen des röntgendichten Abschnitts 120 angeordnet.

**Fig. 5** zeigt eine mögliche Ausgestaltung des röntgendichten Abschnitts 120 in Perspektive. **Fig. 6** zeigt den röntgendichten Abschnitt 120 der Fig. 5 von der Seite.

Der röntgendichte Abschnitt 120 kann einen (oder mehrere) Abschnitte 120b in Form eines Teils einer Kugel aufweisen, der z. B. an einem zylinder- oder stabförmigen Abschnitt 120a befestigt oder hiermit einstückig hergestellt sein kann.

**Fig. 7** zeigt eine mögliche Ausgestaltung eines optional nicht-röntgendichten Löcherabschnitts 200 mit Löchern oder Öffnungen (hier Durchgangsöffnungen) 201 von der Seite, **Fig. 8** von vorne, **Fig. 9** leicht perspektivisch. Die Ausführungsformen der Fig. 7 bis 9 zeigen einen gebogenen Löcherabschnitt 200, z. B. mit einem konstanten Krümmungsradius. Der Löcherabschnitt 200 könnte aber auch gerade oder auf andere Weise gekrümmt verlaufen.

Die Anordnung der Öffnungen 201 ist rein exemplarisch und kann je nach Anwendung unterschiedlich sein. Beispielsweise können über die gesamte Längserstreckung oder nur Teile hiervon Öffnungen 201 regelmäßig oder unregelmäßig verteilt angeordnet sein.

Die Anzahl der Öffnungen 201 beträgt mindestens zwei, kann aber über fünf, über 10 oder mehr betragen.

Der Durchmesser der Öffnungen 201 kann gleich oder unterschiedlich sein. Verschieden große Durchmesser können zur Verbindung mit unterschiedlich großen Röntgenmarkern 100 verwendet werden.

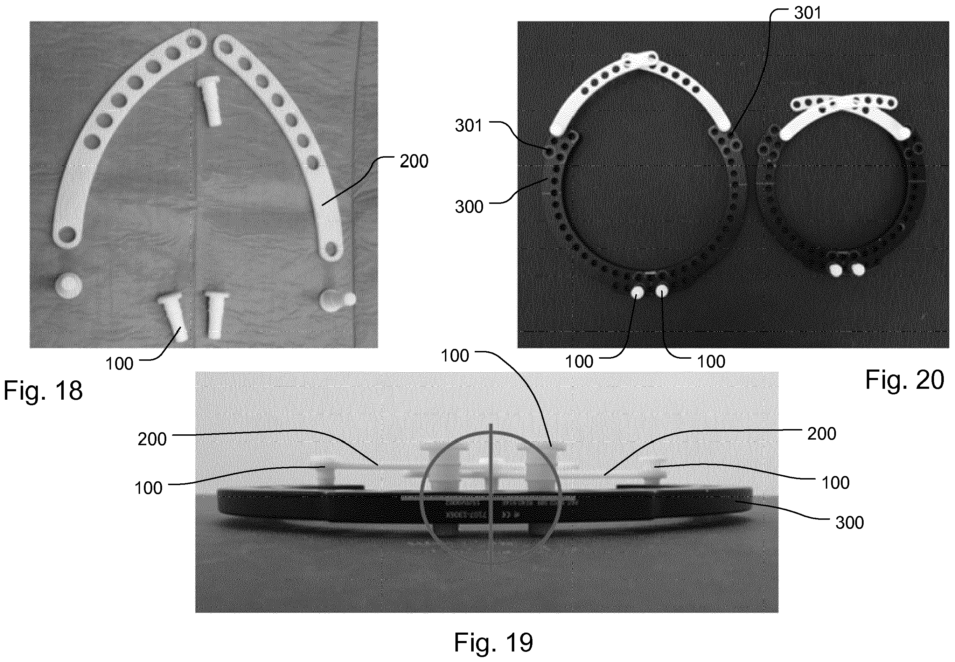

Die exemplarisch als Durchgangsöffnungen bzw. als Durchgangslöcher ausgeführten Öffnungen 201 können als Steckoptionen für den erfindungsgemäße Röntgenmarker 100 verwendet werden. Dies wird beispielsweise in den möglichen Ausführungsformen in Fig. 20 näher beschrieben. Die Öffnungen 201 ermöglichen lösbare Verbindungen der Löcherabschnitte 201 untereinander oder mit einem oder mehreren Ringfixateuren 300.

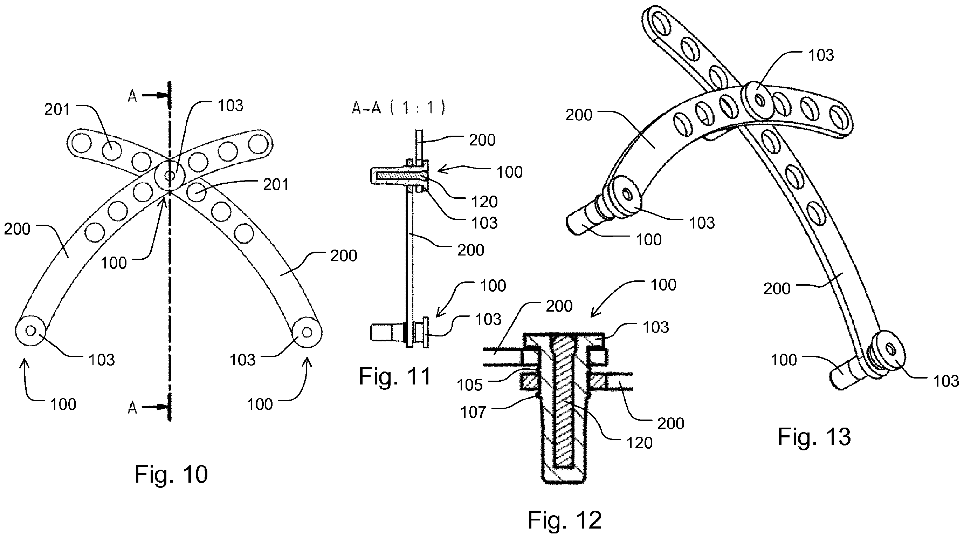

**Fig. 10** zeigt zwei mittels eines erfindungsgemäßen Röntgenmarkers 100 miteinander verbundene Löcherabschnitte 200 in einer Aufsicht.

Der Röntgenmarker 100 ist durch jeweils eine Öffnung 201 eines jeden der beiden Löcherabschnitte 200 durchgesteckt oder in diese eingesteckt. Die auf diese Weise erzielte Verbindung kann optional durch Herausziehen des Röntgenmarkers 100 aus einer oder beiden dieser Öffnungen 201 wieder gelöst werden.

Weiterhin sind in die beiden jeweils an den längsseitigen Enden der beiden Löcherabschnitte 200 gelegenen Öffnungen 201 je ein weiterer Röntgenmarker 100 eingesteckt. Diese beiden Röntgenmarker 100 können insbesondere mit Fixateuren 300 verbunden werden, wie dies in den Fig. 19 und 20 gezeigt und zu diesen näher beschrieben ist.

**Fig. 11** zeigt die beiden miteinander mittels des Röntgenmarkers 100 verbundenen Löcherabschnitte 200 der Fig. 10 in einer Schnittebene A-A in einer Seitenansicht. In der Schnittdarstellung des oberen Röntgenmarkers 100 ist der eingesteckte, röntgendichte Abschnitt 120 erkennbar.

**Fig. 12** zeigt einen (gegenüber der Fig. 11 um 90 Grad gedrehten) Ausschnitt der Fig. 11, der den erfindungsgemäßen Röntgenmarker 100 umfasst.

In dieser Ansicht sind die beiden Lippen 105 und 107 deutlich sichtbar, zwischen welchen einer der beiden Löcherabschnitte 200 eingeschoben, eingeführt oder lösbar fixiert ist.

Der obere Löcherabschnitt 200 ist zwischen die obere Lippe 105 und die Unterkante des Kragens 103 eingeschoben und/oder zwischen diesen fixiert. Die Verbindung des Röntgenmarkers 100 mit den beiden Löcherabschnitten 200 kann durch leichten Zug oder Druck auf die Löcherabschnitte 200 bzw. den Röntgenmarker 100 wieder gelöst werden.

Beide Löcherabschnitte 200 liegen dem Röntgenmarker 100 vorzugweise an optional nicht-konisch ausgestalteten Abschnitten an.

**Fig. 13** zeigt die beiden mit dem erfindungsgemäßen Röntgenmarker 100 verbundenen Löcherabschnitte 200 der Fig. 10 in einer perspektivischen Ansicht.

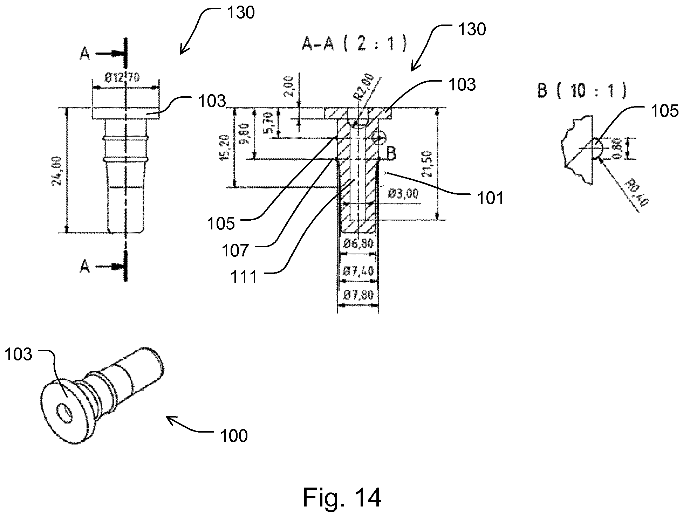

**Fig. 14** zeigt den nicht-röntgendichten Abschnitt 130 des erfindungsgemäßen Röntgenmarkers 100 in verschiedenen Ansichten mit rein exemplarischen Maßangaben. Die Gesamtlänge ist darin exemplarisch 24 mm (bevorzugt zwischen 15 mm und 30 mm), der Außendurchmesser des Kragens 103 beträgt exemplarisch 12,7 mm (bevorzugt zwischen 10 mm und 20 mm) und die Höhe des Kragens 103 beträgt exemplarisch 2 mm (bevorzugt zwischen 1 mm und 4 mm).

Der Abstand der oberen Lippe 105 von dem stirnseitigen Ende mit dem Kragen 103 beträgt exemplarisch 5,7 mm (bevorzugt zwischen 4 mm und 8 mm), der Abstand der unteren Lippe 107 von dem stirnseitigen Ende mit dem Kragen 103 beträgt exemplarisch 9,8 mm (bevorzugt zwischen 5 mm und 15 mm).

Der Abstand zwischen dem Übergang des konischen Abschnitts 101 zum zylindrischen Abschnitt bis zum stirnseitigen Ende mit dem Kragen 103 beträgt exemplarisch 15,2 mm (bevorzugt zwischen 10 mm und 20 mm).

Der konische Abschnitt 101 weitet sich von einem exemplarischen Durchmesser von 6,8 mm (bevorzugt zwischen 4 mm und 9 mm) auf einen Durchmesser von exemplarisch 7,4 mm (bevorzugt zwischen 4 mm und 10 mm) auf.

Der Durchmesser 6,8 mm entspricht dem Durchmesser des zylindrischen Abschnitts, der sich längsseitig an dem Endbereich gegenüber dem Kragen 103 befindet. Der Außendurchmesser, der sich direkt an den Kragen 103 anschließt, beträgt exemplarisch 7,8 mm (bevorzugt zwischen 4 mm und 15 mm).

Die Sacklochbohrung 109 des Innenraums 111 zur Aufnahme des röntgendichten Abschnitts 120 weist exemplarisch eine Länge von 21,5 mm (bevorzugt zwischen 15 mm und 30 mm) und einen Durchmesser von exemplarisch 3 mm (bevorzugt zwischen 1 mm und 6 mm) auf.

Der Radius zur Aufnahme des kugelförmigen Abschnitts 120b des röntgendichten Abschnitts 120 beträgt rein exemplarisch 2 mm (bevorzugt zwischen 1 mm und 6 mm).

Die Form der beiden Lippen 105 und 107, die sich über den gesamten Umfang des nicht-röntgendichten Abschnitts 130 erstrecken können, sind exemplarisch halbkreisförmig mit einem Radius von 0,4 mm (bevorzugt zwischen 0,1 mm und 1 mm) bzw. einem Durchmesser von 0,8 mm (bevorzugt zwischen 0,2 mm und 2 mm).

In der rechten Abbildung der Fig. 14 ist beispielhaft eine 10:1-Vergrößerung des Ausschnitts B mit der Lippe 105 dargestellt.

Die untere Abbildung der Fig. 14 ist eine perspektivische Darstellung des nicht-röntgendichten Abschnitts 130, analog zur Fig. 4.

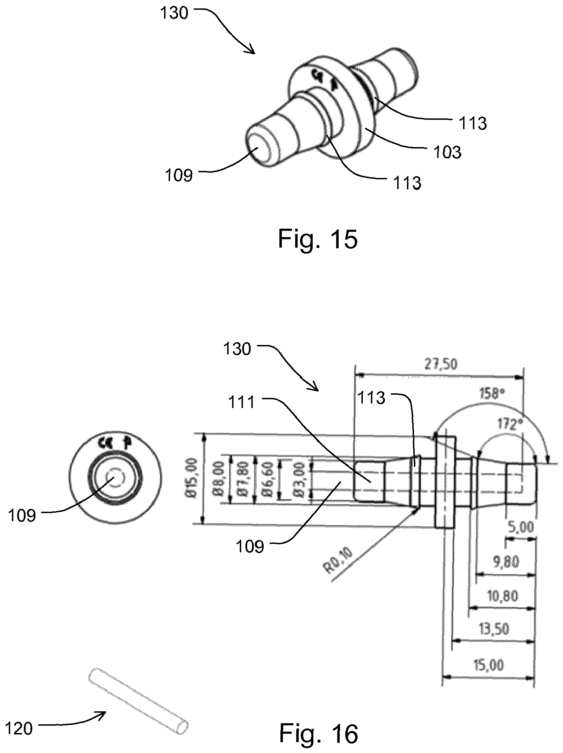

**Fig. 15** zeigt eine weitere optionale Ausgestaltung des nicht-röntgendichten Abschnitts 130 in einer perspektivischen Darstellung.

Diese Ausführungsform ist vorgesehen, um die beiden miteinander zu verbindenden Löcherleisten 200 von jeweils einem der einander in Längsrichtung des Grundkörpers des Röntgenmarkers 100 gelegenen Enden auf den nicht-röntgendichten Abschnitt 130 aufzuschieben.

Der Kragen 103, der hierin als Anschlag dienen und daher auch als solcher bezeichnet werden kann, befindet sich dann zwischen den beiden Löcherleisten 200. Anstelle von zwei Lippen, welche aus z. B. der Fig. 1 mit den Bezugszeichen 105, 107 bezeichnet sind, kann rein optional nur eine Lippe 113, die z. B. ihrerseits als (vorzugsweise sich zum freien Ende des Grundkörpers hin verjüngender) konischer Absatz ausgestaltet sein. Er kann zum axialen Fixieren der jeweiligen Löcherleiste 200 auf dem nicht-röntgendichten Abschnitt 130 ausgestaltet und/oder vorgesehen sein.

Die beiden Längsabschnitte, die sich in jeweils entgegengesetzte Richtung vom Kragen 103 erstrecken, sind rein exemplarisch spiegelbildlich gleich ausgeführt. Alternativ können diese Längsabschnitte unterschiedliche Abmaße aufweisen, beispielsweise, um unterschiedlich dicke Löcherleisten 200 miteinander zu verbinden.

Die Löcherleisten 200 (in Fig. 15 nicht dargestellt) können von beiden Seiten über die konischen Absätze 113 hinweg bis zum als Anschlag dienenden Kragen 103 aufgeschoben werden. Die beiden konischen Absätze 113 dienen anschließend der axialen Fixierung, sodass die Löcherleisten 200 nicht unbeabsichtigt wieder von dem Abschnitt 130 herunterrutschen.

**Fig. 16** zeigt auf der rechten Seite den nicht-röntgendichten Abschnitt 130 der Fig. 15 in einer Seitenansicht mit exemplarischen Bemaßungen. Weiterhin ist eine um 90 Grad gedrehte, stirnseitige Ansicht (links) dargestellt. Unterhalb dieser Ansicht ist eine Ausführungsform eines röntgendichten Abschnitts 120 in einer perspektivischen Ansicht dargestellt. Dieser röntgendichte Abschnitt 120 ist optional zylindrisch ausgestaltet (d. h. optional nur als Stab, ohne Kugelabschnitt) und zum Einschieben in den Innenraum 111 des nicht-röntgendichten Abschnitts 130 vorgesehen. Der Innenraum 111 ist optional als Sackloch ausgeführt.

Die folgenden Maßangaben sind rein exemplarisch als eine mögliche Ausführungsform zu betrachten. Die Gesamtlänge des nicht-röntgendichten Abschnitts 130 beträgt 30 mm (bevorzugt zwischen 20 mm und 40 mm), der Außendurchmesser des Kragens 103 beträgt 15 mm (bevorzugt zwischen 10 mm und 40 mm), die Gesamtbreite des Kragens 103 beträgt 3 mm (bevorzugt zwischen 1 mm und 6 mm).

Die Länge des als Sacklochbohrung ausgeführten Innenraums 111 für den röntgendichten Abschnitt 120 beträgt 27,5 mm (bevorzugt zwischen 15 mm und 40 mm).

Weitere exemplarische Maße sind in Fig. 16 angegeben.

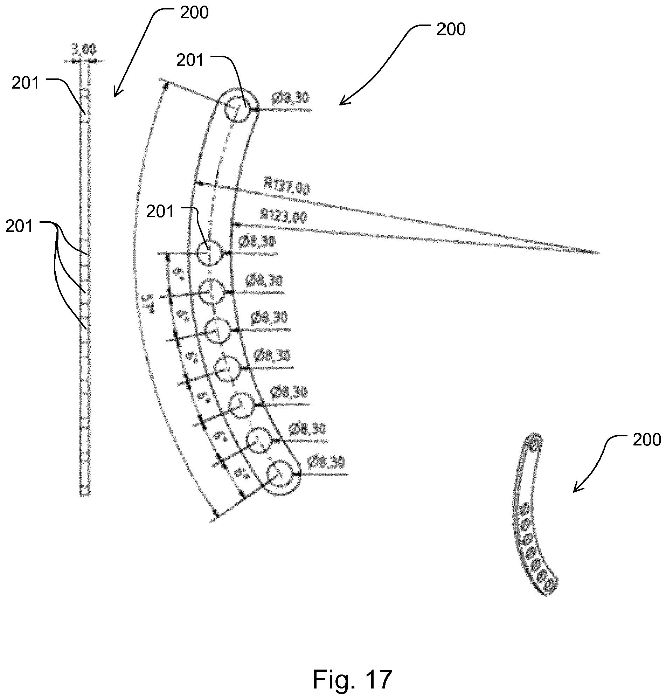

**Fig. 17** zeigt den optional nicht-röntgendichten Löcherabschnitt 200 mit rein exemplarischen Maßangaben in verschiedenen Ansichten.

Beispielsweise beträgt die exemplarische Dicke des Löcherabschnitts 200 3 mm (bevorzugt zwischen 1 mm und 6 mm), der Durchmesser der Öffnungen 201 beträgt exemplarisch 8,3 mm (bevorzugt zwischen 5 mm und 15 mm)und der Umfangswinkel zwischen den beiden äußeren Öffnungen 201, der als Umfangswinkel zwischen den beiden Lochkreisen bezeichnet wird, beträgt exemplarisch 57 Grad (bevorzugt zwischen 45 Grad und 70 Grad). Der äußere Krümmungsradius des Löcherabschnitts 200 beträgt exemplarisch 137 mm (bevorzugt zwischen 100 mm und 150 mm), der innere Krümmungsradius exemplarisch 123 mm (bevorzugt zwischen 90 mm und 140 mm). Weitere exemplarische Maße sind in Fig. 17 angegeben.

**Fig. 18** zeigt zwei Löcherabschnitte 200 und fünf erfindungsgemäße Röntgenmarker 100 als Einzelteile vor einem Zusammenbau für eine Anwendung bei einer chirurgischen Operation an einem Bein. Dies wird in den folgenden Figuren näher beschrieben.

Die in Fig. 18 gezeigten Strukturen ergeben zusammen eine Ausführungsform eines erfindungsgemäßen Kits.

**Fig. 19** zeigt einen Ringfixateur 300, oder Abschnitte hiervon, der mit den Einzelteilen der Fig. 18 verbunden ist, in einer Seitenansicht.

In dieser Ansicht wird deutlich, wie die einzelnen erfindungsgemäßen Röntgenmarker 100 die Löcherabschnitte 200 miteinander verbinden und die Löcherabschnitte 200 wiederum mit dem Ringfixateur 300 verbinden.

Die beiden vorderen, nach oben herausstehenden Röntgenmarker 100 können beispielsweise zum Befestigen weiterer Teile, insbesondere am Ringfixateur 300, und/oder zur Positionserkennung oder -ermittlung in einem Röntgenbild genutzt werden.

Das dargestellte, optionale Fadenkreuz kann zur Orientierung der Anordnung und zur Bestimmung eines Mittelpunktes bzw. Referenzpunktes genutzt werden.

**Fig. 20** zeigt eine Aufsicht von zwei unterschiedlich großen, nebeneinander angeordneten Ringfixateuren 300, die jeweils mit Löcherabschnitten 200 und Röntgenmarkern 100 verbunden sind. Unterschiedliche Größen von Ringfixateuren 300 können zur Anordnung und Fixierung mehrerer Knochensegmente oder Frakturteile verwendet werden.

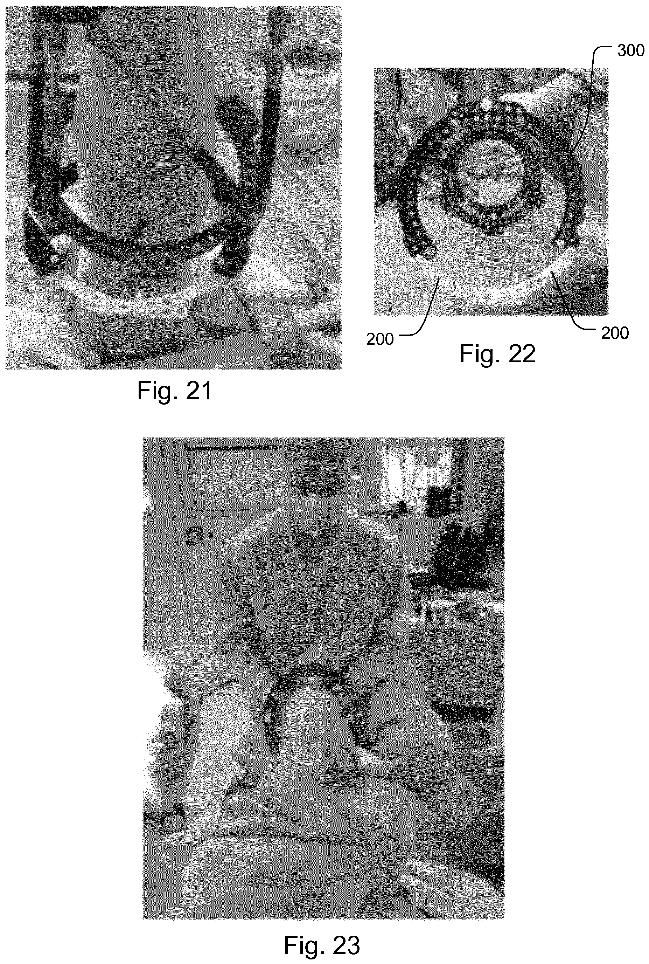

**Fig. 21** zeigt eine exemplarische, chirurgische Anwendung an einem Bein eines Patienten mit einer Anordnung von zwei Ringfixateuren 300, Löcherabschnitten 200 und erfindungsgemäßen Röntgenmarkern 100. Die Ringfixateure 300 sind mit weiteren Fixierungen und Halterungen verbunden, um beispielsweise eine Fraktur zu fixieren.

**Fig. 22** zeigt drei hintereinander angeordnete Ringfixateure 300, wobei der vorderste zwei Löcherabschnitte 200 und mehrere Röntgenmarker 100 aufweist.

**Fig. 23** zeigt eine weitere Anordnung von Ringfixateuren 300 während einer Beinoperation.

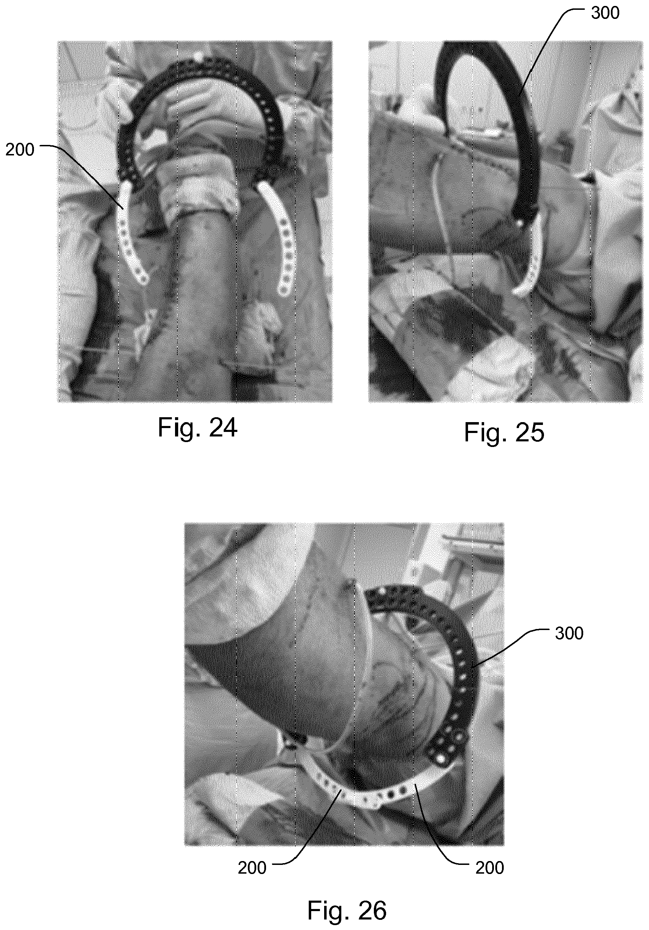

**Fig. 24** zeigt das Anpassen eines Ringfixateurs 300 an ein Bein eines Patienten während einer Operation, wobei die Löcherabschnitte 200 noch nicht mittels erfindungsgemäßer Röntgenmarker 100 miteinander verbunden sind.

**Fig. 25** zeigt das Anpassen des Ringfixateurs 300 der Fig. 24 in einem zweiten Schritt.

**Fig. 26** zeigt das Anpassen des Ringfixateurs 300 der Fig. 24 und 25 in einem dritten Schritt, wobei die Löcherabschnitte 200 mittels erfindungsgemäßer Röntgenmarker 100 miteinander verbunden sind.

### Bezugszeichenliste

- 100: Röntgenmarker
- 101: konischer Abschnitt
- 103: Kragen
- 105: Lippe, Ring
- 107: Lippe, Ring
- 109: Öffnung
- 111: Innenraum
- 113: konischer Absatz
- 120: röntgendichter Abschnitt des Röntgenmarkers
- 120a: (zylinder- oder stabförmiger) Abschnitt des röntgendichten Abschnitts
- 120b: (kugelförmiger) Abschnitt des röntgendichten Abschnitts
- 130: nicht-röntgendichter Abschnitt des Röntgenmarkers
- 200: Löcherabschnitt, Löcherleiste (nicht röntgendicht)
- 201: Öffnungen, Durchgangsöffnungen
- 300: Ringfixateur
- 301: Öffnungen des Ringfixateurs

## Patentansprüche

1. Röntgenmarker (100) für ein medizinisches Instrument, insbesondere zum Einstecken in Öffnungen (301) eines Ringfixateurs (300), aufweisend einen röntgendichten Abschnitt (120).

2. Röntgenmarker (100) nach Anspruch 1, wobei der röntgendichte Abschnitt (120) einen zylindrischen Abschnitt (120a) aufweist.

3. Röntgenmarker (100) nach einem der vorhergehenden Ansprüche, wobei der röntgendichte Abschnitt (120) wenigstens einen Abschnitt (120b) in Form einer Kugel oder eines Kugelteilabschnitts aufweist.

4. Röntgenmarker (100) nach einem der vorhergehenden Ansprüche, weiter aufweisend einen nicht-röntgendichten Abschnitt (130).

5. Röntgenmarker (100) nach Anspruch 4, wobei der nicht-röntgendichte Abschnitt (130) den röntgendichten Abschnitt (120) teilweise oder vollständig umgibt.

6. Röntgenmarker (100) nach einem der vorhergehenden Ansprüche, wobei der nicht-röntgendichte Abschnitt (130) und/oder der röntgendichte Abschnitt (120) zumindest abschnittsweise konisch zuläuft.

7. Röntgenmarker (100) nach einem der vorhergehenden Ansprüche, aufweisend einen Kragen (103), der, falls vorgesehen, über den konisch zulaufenden Abschnitt radial, also senkrecht zu einer Längsachse des Röntgenmarkers (100), übersteht.

8. Röntgenmarker (100) nach einem der vorhergehenden Ansprüche, aufweisend wenigstens eine Lippe (105, 107), die den Röntgenmarker (100) oder einen Grundkörper hiervon nach radial abschließt.

9. Röntgenmarker (100) nach Anspruch 8, wobei die Lippe (105, 107) in Umfangsrichtung des Röntgenmarkers (100) geschlossen ist.

10. Röntgenmarker (100) nach einem der vorhergehenden Ansprüche, dessen radiale Durchmesser in einem Bereich liegen, die zwischen 5 mm und 20 mm betragen, vorzugsweise zwischen 5 mm und 15 mm, besonders bevorzugt zwischen 7 mm und 12 mm.

11. Kit mit
- wenigstens einem oder mehreren Röntgenmarkern (100) nach einem der vorangegangenen Ansprüche;
- wenigstens einer Löcherleiste (200), vorzugsweise aus einem nicht-röntgendichten Material oder ein solches aufweisend;
wobei die Löcherleiste (200) eine Vielzahl von Sacklöchern oder Durchgangslöchern (201) aufweist, deren Durchmesser es vorzugsweise erlauben, einen der Röntgenmarker (100) zumindest abschnittsweise aufzunehmen.

12. Kit nach Anspruch 11, weiter aufweisend
- wenigstens einen Ringfixateur (300) mit der Form eines Teils eines Kreises oder eines Teils einer Ellipse, aufweisend eine Vielzahl von Sacköffnungen oder Durchgangsöffnungen (301);
wobei die Sacklöcher oder Durchgangslöcher (301) des Ringfixateurs (300) vorzugsweise einen Durchmesser aufweisen, der es erlaubt, einen der Röntgenmarker (100) zumindest abschnittsweise aufzunehmen.
